(19) Europäisches Patentamt
European Patent Office
Office européen des brevets

(11) Veröffentlichungsnummer: **0 236 994**
**B1**

(12) # EUROPÄISCHE PATENTSCHRIFT

(45) Veröffentlichungstag der Patentschrift: **20.12.89**

(51) Int. Cl.⁴: **C07D 233/04**, C07D 239/06, C07D 243/04, C07D 245/02

(21) Anmeldenummer: **87103284.3**

(22) Anmeldetag: **07.03.87**

(54) **Verfahren zur Herstellung von Acryl- und Methycrylsäureestern.**

(30) Priorität: **11.03.86 DE 3607995**

(43) Veröffentlichungstag der Anmeldung: **16.09.87 Patentblatt 87/38**

(45) Bekanntmachung des Hinweises auf die Patenterteilung: **20.12.89 Patentblatt 89/51**

(84) Benannte Vertragsstaaten: **BE DE FR GB IT NL**

(56) Entgegenhaltungen:
**US-A- 2 871 223**

**CHEMICAL ABSTRACTS, Band 102, Nr. 5, 4. Februar 1985, Columbus, Ohio, USA NATIONAL STARCH AND CHEMICAL CORP: "Polymerizable Imidazolidinones" Seite 578, Spalte 1, Zusammenfassung Nr. 45 947e**
**CHEMICAL ABSTRACTS, Band 81, Nr. 14, 7. Oktober 1974, Columbus, Ohio, USA PORRET, DANIEL, HABERMEIER JUERGEN "Diazaheterocycle-containing acrylic acid esters and their polymerization" Seite 16, Spalte 2, Zusammenfassung Nr. 78 465x**

(73) Patentinhaber: **BASF Aktiengesellschaft, Carl-Bosch-Strasse 38, D-6700 Ludwigshafen(DE)**

(72) Erfinder: **Merger, Franz, Dr., Max-Slevogt-Strasse 25, D-6710 Frankenthal(DE)**
Erfinder: **Hutmacher, Hans-Martin Dr., Ruedigerstrasse 70, D-6700 Ludwigshafen(DE)**
Erfinder: **Schwarz, Wolfgang, Dr., Wesostrasse 132, D-7507 Pfinztal(DE)**
Erfinder: **Nestler, Gerhard, Dr., Van-Leyden-Strasse 17, D-6700 Ludwigshafen(DE)**
Erfinder: **Szucsanyi, Maria Gyopar, Dr., Rene-Bohn-Strasse 4, D-6700 Ludwigshafen(DE)**
Erfinder: **Mueller-Mall, Rudolf, Dr., Ulmenweg 39, D-6708 Neuhofen(DE)**

**EP 0 236 994 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein neues, verbessertes Verfahren zur Herstellung von Acryl- oder Methacrylsäureestern der Formel I

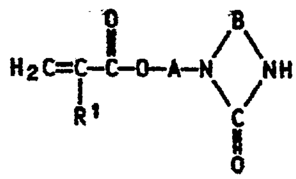

I

in der $R^1$ Wasserstoff oder eine Methylgruppe darstellt und A und B für eine verzweigte oder unverzweigte Alkylengruppe mit 2 bis 5 Kohlenstoffatomen stehen.

Diese Verbindungen sind interessante Comonomere z.B. für die Herstellung von Anstrichdispersionen oder von Lederhilfsmitteln (z.B. US-PS 2 828 224 oder 3 356 627). Sie werden z.B. nach dem in der US-PS 2 871 223 beschriebenen Verfahren ausgehend von Acryl- oder Methacrylsäurechlorid und Umesterung mit Imidazolidin-2-on-derivaten in Gegenwart eines tertiären Amins oder Pyridin erhalten. Dabei fallen jedoch stöchiometrische Mengen an Ammoniumchloriden oder Pyridiniumhydrochlorid an, die vor der Weiterverarbeitung der Produkte abgetrennt werden müssen. Dadurch ist das Verfahren technisch aufwendig und für den technischen Maßstab praktisch ungeeignet. Außerdem entstehen beträchtliche Mengen an doppelsubstituierten Nebenprodukten.

Der Erfindung lag daher die Aufgabe zugrunde, ein im technischen Maßstab durchführbares einfaches und selektives Verfahren für die Herstellung von I zu finden, nach dem Produkte erhalten werden, die ohne aufwendige Reinigungsoperationen als Komponenten für Dispersionen verwendbar sind.

Demgemäß wurde ein Verfahren gefunden, nach dem Acryl- oder Methacrylsäureester der Formel I

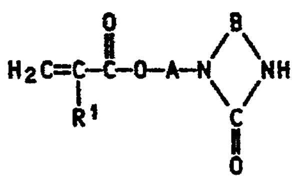

I

in der $R^1$ Wasserstoff oder eine Methylgruppe darstellt und A und B für eine verzweigte oder unverzweigte Alkylengruppe mit 2 bis 5 Kohlenstoffatomen stehen, vorteilhaft hergestellt werden, wenn man ein Acrylat oder Methacrylat der Formel II

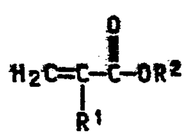

II,

in der $R^2$ einen Alkylrest mit 1 bis 4 Kohlenstoffatomen bedeutet, mit einem Heterocyclus der Formel III

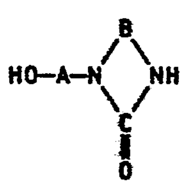

III

in Gegenwart von Titanalkoholaten oder Chelatverbindungen der Metalle Titan, Zirkonium, Eisen oder Zink mit 1,3-Dicarbonylverbindungen umsetzt.

Der Erfolg des Verfahrens ist überraschend, da zu erwarten war, daß aufgrund des bifunktionellen Charakters von I leicht Folgereaktionen mit II auftreten würden, die der unmittelbaren Verwendung von I für Dispersionen entgegenstehen würden. Als Folge-bzw. Konkurrenzreaktion zur erfindungsgemäßen Umsetzung waren insbesondere basenkatalysierte Michael-Additionen zu erwarten. Solche Additionen von Acrylaten bzw. Methacrylaten an Harnstoffderivate z.B. die in der US-PS 4 211 804 verwendeten Diisocyanate oder an Benzimidazolonderivate wie in der DE-OS 25 27 261 (Anspruch 10c und Beispiel 25) und der DE-AS 1 545 997 beschrieben sowie an Pyrimidin-Derivaten (s. z.B. Tetrahedron Letters, No.53, S. 4605-4606) sind allgemein bekannt. Beispielsweise sind bei der Umesterung von Methacrylsäureestern mit 1-(2-Hydroxyethyl)-imidazolidin-2-on leicht Folgereaktionen der gebildeten 1-(2-Methacroyloxyethyl)-imidazolidin-2-one zu 1-(2-Methacroyloxyethyl)-3-(2-carbalkoxypropyl)-imidazolidin-2-onen (Michaelprodukte) und/oder 1-(2-Methacroyloxyethyl)-3-methacroyl-imidazolidin-2-on möglich. Solche Folgereaktionen werden z.B. bei der Umesterung in Gegenwart gängiger Katalysatoren wie Natriummethylat, Kaliumcarbonat, Kaliumhydroxid oder 1,8-Diazabicyclo[5.4.0]undec-7-en (DBU) beobachtet (s. Vergleichsbeispiel 6). Eine Abtrennung ist aufgrund der Thermolabilität und Polymerisationsfreudigkeit des Produktes im technischen Maßstab nicht möglich.

Bei Verwendung von Methylmethacrylat und 1-(2-Hydroxyethyl)-imidazolidin-2-on läßt sich die Umsetzung durch folgende Reaktionsgleichung wiedergeben:

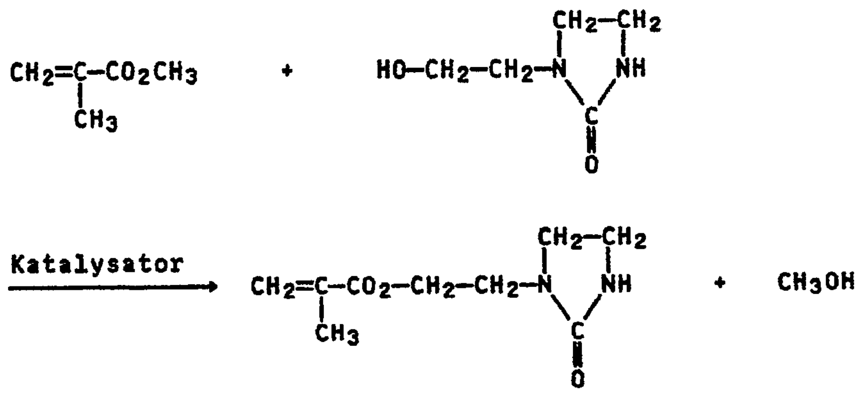


Für das erfindungsgemäße Verfahren können Acryl- oder Methacrylsäureester II verwendet werden, in denen $R^1$ einen Alkylrest mit 1 bis 4, vorzugsweise 1 bis 2 Kohlenstoffatomen bedeutet. Beispielsweise seien Ethyl-, Propyl-, Butyl-, i-Butyl und insbesondere Methylmethacrylat oder -acrylat genannt.

Als Ausgangsstoffe III kommen solche Verbindungen in Frage, in denen A oder B eine verzweigte oder unverzweigte Alkylengruppe mit 2 bis 5 Kohlenstoffatomen darstellt, z.B. $-C_2H_4-$, $-CH(CH_3)CH_2-$, $CH_2CH(CH_3)-$, $-(CH2)_3-$, $-(CH_2)_5-$, $-CH_2CH(CH_3)CH_2-$, $-CH_2C(CH_3)_2CH_2-$. Vorzugsweise beträgt die Ringgliederzahl des Heterocyclus 5 und 6. Besonders vorteilhaft wird 1-(2-Hydroxyethyl)-imidazolidin-2-on, das gut aus Aminoethylethanolamin und Harnstoff z.B. nach US-PS 3 254 075 zugänglich ist, verwendet.

Als Katalysatoren für die Umesterung kommen erfindungsgemäß Titanalkoholate oder Chelate der Metalle Eisen, Zink, Titan und/oder Zirkonium mit 1,3-Dicarbonylverbindungen in Betracht.

Bei den Alkoholaten des Titans können als Alkoholkomponente Alkohole mit 1 bis 8, insbesondere 2 bis 4 Kohlenstoffatomen eingesetzt werden, z.B. Ethanol, Propanol, iso-Propanol, Allylalkohol, n-Butanol und iso-Butanol. Die Darstellungsweise dieser Verbindungen ist z.B. in Houben-Weyl, Methoden der organischen Chemie, 4. Auflage, Bd. VI/2, 1963, S. 21-26 beschrieben.

Besonders geeignete Umesterungskatalysatoren sind Tetraalkyltitanate, z.B. Tetramethyl-, Tetrapropyl-, Tetraisopropyl- oder Tetra-n-butyltitanat.

Bei den Katalysatoren, bestehend aus Chelaten der oben genannten Metalle mit 1,3-Dicarbonylverbindungen, sind normalerweise soviele Molekeln Dicarbonylverbindung gebunden wie der Oxidationsstufe des Metalls entspricht. Als Chelatbildner kommen 1,3-Dicarbonylverbindungen wie z.B. Acetessigester, Acetylaceton, 3-Methylacetylaceton (3-Methyl-pentandion-2,4), Benzoylaceton oder Dibenzoylmethan in Betracht, besonders geeignet sind Metallchelate von 1,3-Diketonen, insbesondere Acetylacetonate. Die Herstellung der Metallchelate und ihre Verwendung ist z.B. in Houben-Weyl, Methoden der organischen Chemie, 4. Auflage, Bd. VI/2, 1963, Seiten 53-55 und 58-61 oder A.E. Martell, M. Calvin, Die Chemie der Metallchelatverbindungen (1958) beschrieben.

Die Katalysatoren werden im allgemeinen einzeln oder im Gemisch in Mengen von 0,01 bis 10 Mol.%, bezogen auf III eingesetzt. Die Zugabe größerer Mengen ist möglich, wirtschaftlich im allgemeinen jedoch nicht erforderlich. Im Fall der Acetylacetonate sind 0,05 bis 1,0 Mol.% im Fall der Tetra-$C_2$-$C_4$-alkyltitanate 0,2 bis 10 Mol.% vorteilhaft.

Die Ester II können in Mengen von 1 bis 20, vorzugsweise 2 bis 10, insbesondere 3 bis 6 mol pro Mol III eingesetzt werden.

Die Umsetzung wird zweckmäßigerweise in Gegenwart von üblichen Polymerisationsinhibitoren wie z.B. Phenothiazin oder Hydrochinonmonomethylether, insbesondere in Gegenwart von Sauerstoff durchgeführt. Der Sauerstoff wird in der Regel in Form von Luft und in solchen Mengen zugesetzt, daß der Gehalt in der Gasphase über dem Reaktionsgemisch unterhalb der Explosionsgrenze bleibt. Beispielsweise haben sich Mengen von 0,1 bis 1 l pro Stunde und Mol cyclischen Harnstoff bewährt.

Die Umsetzung kann unter Normaldruck, Unter-oder Überdruck durchgeführt werden. Geeignete Reaktionstemperaturen sind 30 bis 150, bevorzugt 50 bis 130, insbesondere 70 bis 120°C. Die Umsetzung kann diskontinuierlich oder kontinuierlich erfolgen. Zweckmäßigerweise kann man so verfahren, daß

man die Ausgangsstoffe II und III gemeinsam zum Sieden erhitzt und dabei kontinuierlich das freiwerdende Alkanol, gegebenenfalls in Form seines Azeotrops mit dem Ester II, abdestilliert. Die Reaktionszeiten liegen je nach Reaktionstemperatur und Katalysator bei ca. 1 bis 6 Stunden. Es ist auch möglich, die Umsetzung in Gegenwart eines inerten Lösungsmittels, z.B. Toluol oder Cyclohexan, durchzuführen.

Nach Beendigung der Reaktion kann der Katalysator, falls erforderlich, in an sich bekannter Weise abgetrennt werden. Tetraalkyltitanate lassen sich z.B. nach Hydrolyse mit Wasser durch Filtrieren oder Zentrifugieren abtrennen.

Bei Verwendung von Zirkonium-2,4-pentandionat kann überraschend besonders vorteilhaft auf das Abtrennen des Katalysators verzichtet werden, ohne daß die anwendungstechnischen Eigenschaften beim Einsatz der Produkte in Acrylatdispersionen beeinträchtigt werden.

Das Produkt kann aus den Reaktionsgemischen in üblicher Weise, z.B. durch Abdampfen des überschüssigen Esters II isoliert werden. Es ist jedoch besonders zweckmäßig und wirtschaftlich, die Umsetzung mit einem (Meth)-acrylsäureester durchzuführen, der für den späteren Einsatz in Acrylatdispersionen nicht bzw. nicht vollständig abgetrennt werden muß, d.h. mit einpolymerisiert werden kann.

Bei Umsätzen über 90 % kann häufig ohne Qualitätseinbußen ebenfalls auf die Abtrennung entsprechender Anteile an unumgesetzter Hydroxiverbindung III verzichtet werden.

Im Vorzugsbereich fallen 20 bis 50 %ige Produktlösungen an, die direkt in Acrylatdispersionen einpolymerisiert werden können.

## Beispiel 1

2 700g Methylmethacrylat wurden zusammen mit 780 g 1-(2-Hydroxyethyl)-imidazolidin-2-on, 13 g Tetraethyltitanat und 2,7g Phenothiazin unter Rühren und Einleiten von stündlich 0,9 l Luft zum Sieden erhitzt. Über eine Füllkörperkolonne wurden dabei in 5,3 Stunden 212 g des Azetrops von Methanol mit Methylacrylat vom Siedepunkt 65°C abdestilliert.

Nach Beendigung der Umsetzung wurden zur Abtrennung des Katalysators nach Abkühlen auf 25°C 175 g Wasser zugegeben und der dabei ausfallende Niederschlag nach 0,5 Stunden durch Zentrifugieren abgetrennt (69 g).

Man erhielt 3 387g einer Lösung, die nach quantitativer HPLC-Analyse 31,9 % 1-(2-Methacroyloxyethyl)-imidazolidin-2-on entsprechend einer Ausbeute von 91 %, bezogen auf eingesetztes 1-(2-Hydroxyethyl)-imidazolidin-2-on, enthielt.

## Beispiel 2

130 g 1-(2-Hydroxyethyl)-imidazolidin-2-on, 450g Methylmethacrylat, 0,7 g Zirkoniumacetylacetonat und 0,1 g Phenothiazin wurden unter Rühren und Einleiten von stündlich 0,9 l Luft zum Sieden erhitzt. Wie in Beispiel 1 wurden innerhalb von 2,6 Stunden 36 g des Azeotrops von Methanol mit Methylmethacrylat abdestilliert.

Es blieben 544 g Produktlösung zurück, die nach quantitativer HPLC-Analyse 33,4 % 1-(2-Methacroyloxyethyl)-imidazolidin-2-on entsprechend einer Ausbeute von 91,5 % bezogen auf eingesetztes 1-(2-Hydroxyethyl)- imidazolidin-2-on enthielten. Bei 93,7 % Umsatz entspricht dies einer Selektivität von 97,8 %.

## Beispiel 3

130 g 1-(2-Hydroxyethyl)-imidazolidin-2-on, 450 g Methylmethacrylat, 0,7 g Zinkacetylacetonat und 0,1 g Phenothiazin wurden unter Rühren und Einleiten von stündlich 0,9 l Luft zum Sieden erhitzt. Wie in Beispiel 1 wurden in 3,5 Stunden 40 g des Azeotrops von Methanol mit Methylmethacrylat abdestilliert.

Es blieben 540 g Produktlösung zurück, die nach quantitativer HPLC-Analyse 27,7 % 1-(2-Methacroyloxyethyl)-imidazolidin-2-on entsprechend einer Ausbeute von 75,6 % bezogen auf eingesetztes 1-(2-Hydroxyethyl)-imidazolidin-2-on enthielten. Bei 85,5% Umsatz entspricht dies einer Selektivität von 88,4 %.

## Beispiel 4

130 g 1-(2-Hydroxyethyl)-imidazolidin-2-on, 450 g Methylmethacrylat, 3,5 g Eisenacetylacetonat und 0,1 g Phenothiazin wurden unter Rühren und Einleiten von stündlich 0,9 l Luft zum Sieden erhitzt. Wie in Beispiel 1 wurden innerhalb von 5,3 Stunden 32 g des Azeotrops von Methanol mit Methylmethacrylat abdestilliert.

Es blieben 550 g Produktlösung zurück, die nach quantitativer HPLC-Analyse 22,1 % 1-(2-Methacroyloxyethyl)-imidazolidin-2-on entsprechend einer Ausbeute von 61,4 % bezogen auf eingesetztes 1-(2-Hydroxyethyl)-imidazolidin-2-on enthielten. Bei 68,3 % Umsatz entspricht dies einer Selektivität von 89,9 %.

Beispiel 5

1 454,6 g Aminoethylethanolamin un 840 g Harnstoff wurden nach Verdrängen der überstehenden Luft mit Stickstoff in 0,5 Stunden auf 130°C und danach während 3,5 Stunden bis auf 210°C erhitzt. Nach weiteren 0,5 Stunden Nachheizen bei 210°C war die unter Abspaltung von Ammoniak ablaufende Reaktion beendet.

Man erhielt 1 815g eines Produkts, das nach quantitativer HPLC-Analyse 96,6 % 1-(2-Hydroxyethyl)-imidazolidin-2-on enthielt, entsprechend einer Ausbeute von 96,3 %.

134,9 g dieses Materials wurden ohne weitere Reinigung mit 450 g Methylmethacrylat, 0,49 g Zirkoniumacetylacetonat und 0,1 g Phenothiazin unter Rühren und Einleiten von 0,9 l Luft zum Sieden erhitzt. Wie in Beispiel 1 wurden in 4,1 Stunden 37,6 g des Azeotrops von Methanol mit Methylmethacrylat abdestilliert.

Es blieben 547 g Produktlösung zurück, die nach quantitativer HPLC-Analyse 33,2 % 1-(2-Methacroyloxyethyl)-imidazolidin-2-on enthielten, was einer Ausbeute von 91,8 % bezogen auf eingesetztes 1-(2-Hydroxyethyl)-imidazolidin-2-on entspricht. Bei 93,3 % Umsatz entspricht dies einer Selektivität von 98,4 %.

Vergleichsversuch 6

130 g 1-(2-Hydroxyethyl)-imidazolidin-2-on, 450 g Methylmethacrylat, 1,8 g 30 %ige methanolische Natriummethylatlösung und 0,1 g Phenothiazin wurden unter Rühren zum Sieden erhitzt. Wie in Beispiel 1 wurden in 0,6 Stunden 38,1 g des Azeotrops von Methanol mit Methylmethacrylat abdestilliert.

Das Reaktionsgemisch enthielt nach gaschromatographischer Analyse 1-(2-Methacroyloxyethyl)-3-(2-carbmethoxypropyl)-imidazolidin-2-on (Nebenprodukt) und 1-(2-Methacroyloxyethyl)-imidazolidin-2-on (Wertprodukt) im Verhältnis 4,3:1.

**Patentansprüche**

1. Verfahren zur Herstellung von Acryl- oder Methacrylsäureestern der Formel I

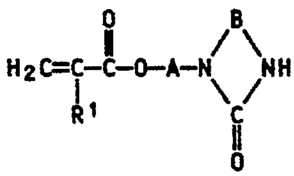

I

in der $R^1$ Wasserstoff oder eine Methylgruppe darstellt und A und B für eine verzweigte oder unverzweigte Alkylengruppe mit 2 bis 5 Kohlenstoffatomen steht, dadurch gekennzeichnet, daß man ein Acrylat oder Methacrylat der Formel II

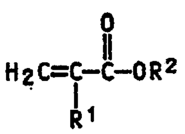

II,

in der $R^2$ einen Alkylrest mit 1 bis 4 Kohlenstoffatomen bedeutet, mit einem Heterocyclus der Formel III

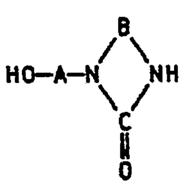

III

in Gegenwart von Titanalkoholaten oder Chelatverbindungen der Metalle Titan, Zirkonium, Eisen oder Zink mit 1,3-Dicarbonylverbindungen umsetzt.

2. Verfahren nach Anspruch 1, dadurch gekennzeichnet, daß man die Umsetzung in Gegenwart von Tetraalkyltitanaten durchführt.

3. Verfahren nach Anspruch 1, dadurch gekennzeichnet, daß man die Umsetzung in Gegenwart von Acetylacetonaten des Zirkonium, Zink, Titan oder Eisen durchführt.

4. Verfahren nach den Ansprüchen 1 bis 3, dadurch gekennzeichnet, daß man Methyl- oder Ethylmethacrylat mit 1-(2-Hydroxyethyl)-imidazolidin-2-on umsetzt.

5. Verfahren nach den Ansprüchen 1 bis 4, dadurch gekennzeichnet, daß man während der Reaktion das freiwerdende Alkanol des Esters II kontinuierlich aus dem Reaktionsgemisch entfernt.

6. Verfahren nach den Ansprüchen 1 bis 5, dadurch gekennzeichnet, daß man die Umsetzung in Gegenwart von Polymerisationsinhibitoren durchführt.

7. Verfahren nach Anspruch 6, dadurch gekennzeichnet, daß man die Umsetzung in Gegenwart von Luftsauerstoff durchführt.

## Claims

1. A process for preparing an acrylate or methacrylate of the formula I

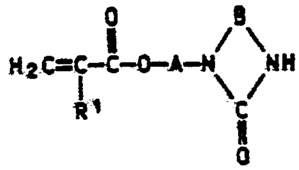

I

where $R^1$ is hydrogen or methyl and A and B are each branched or unbranched alkylene of 2 to 5 carbon atoms, which comprises reacting an acrylate or methycrylate of the formula II

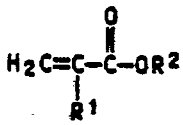

II,

where $R^2$ is alkyl of 1 to 4 carbon atoms with a heterocycle of the formula III

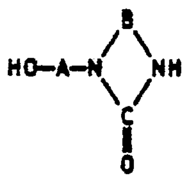

III

in the presence of a titanium alcoholate or a chelate compound of one of the metals titanium, zirconium, iron or zinc with a 1,3-dicarbonyl compound.

2. A process as claimed in claim 1, wherein the reaction is carried out in the presence of a tetraalkyltitanale.

3. A process as claimed in claim 1, wherein the reaction is carried out in the presence of an acetylacetonate of zirconium, zinc, titanium or iron.

4. A process as claimed in claims 1 to 3, wherein methyl or ethyl methacrylate is reacted with 1-(2-hydroxy-ethyl)-imidazolidin-2-one.

5. A process as claimed in claims 1 to 4, wherein the alkanol of ester II, which is eliminated in the course of the reaction, is continuously removed from the reaction mixture.

6. A process as claimed in claims 1 to 5, wherein the reaction is caried out in the presence of a polymerization inhibitor.

7. A process as claimed in claim 6, wherein the reaction is carried out in the presence of atmospheric oxygen.

## Revendications

1. Procédé de préparation d'esters d'acide acrylique ou méthacrylique de formule I

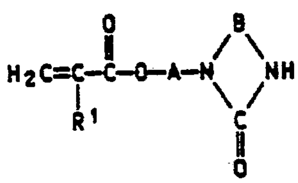

I

dans laquelle $R^1$ représente un atome d'hydrogène ou un groupement méthyle et A et B représentent un groupement alkylène ramifié ou non ramifié ayant de 2 à 5 atomes de carbone, caractérisé en ce qu'on fait réagir un acrylate ou un méthacrylate de formule II

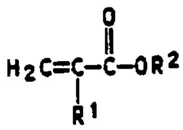

II,

dans laquelle $R^2$ représente un groupement alkyle ayant de 1 à 4 atomes de carbone, avec un hétérocycle de formule III

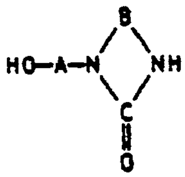

III

en présence d'alcoolates de titane ou de chélates des métaux titane, zirconium, fer ou zinc avec des composés 1,3-dicarbonylés.

2. Procédé selon la revendication 1, caractérisé en ce qu'on mène la réaction en présence de titanates de tétra-alkyle.

3. Procédé selon la revendication 1, caractérisé en ce qu'on mène la réaction en présence d'acétyl-acétonates de zirconium, de zinc, de titane ou de fer.

4. Procédé selon l'une des revendications 1 à 3, caractérisé en ce qu'on fait réagir le méthacrylate de méthyle ou d'éthyle avec la 1-(2-hydroxyéthyl)-imidazolidine-2-one.

5. Procédé selon l'une des revendications 1 à 4, caractérisé en ce que, pendant la réaction, on enlève continuellement du mélange réactionnel l'alcool libéré de l'ester II.

6. Procédé selon l'une des revendications 1 à 5, caractérisé en ce qu'on mène la réaction en présence d'inhibiteurs de polymérisation.

7. Procédé selon la revendication 6, caractérisé en ce qu'on mène la réaction en présence d'oxygène de l'air.